# FASCICULE DE BREVET EUROPEEN

(11) **EP 2 983 623 B1**
(45) Date de publication et mention de la délivrance du brevet: **22.03.2017**
(21) Numéro de dépôt: 14726675.3
(22) Date de dépôt: 10.04.2014
(51) Int. Cl.: A61F 2/46, A61B 17/00, A61F 2/30

(54) **MANCHE AMOVIBLE MUNI D'UN DISPOSITIF D'ASSEMBLAGE DÉMONTABLE**
ABNEHMBARER GRIFF MIT EINER VORRICHTUNG FÜR LÖSBARE MONTAGE
REMOVABLE HANDLE PROVIDED WITH A DEVICE FOR DETACHABLE ASSEMBLY

(30) Priorité: 11.04.2013 FR 1353260
(43) Date de publication de la demande: 17.02.2016
(73) Titulaire: Etablissements Maurice Marle, 52800 Nogent (FR)
(72) Inventeur: MUGNIER, Lionel, F-52800 Vitry Les Nogent (FR)
(74) Mandataire: Oudin, Stéphane
(86) Numéro de dépôt international: PCT/FR2014/050878
(87) Numéro de publication internationale: WO 2014/167258

(56) Documents cités:
- EP-A2- 1 832 248
- FR-A1- 2 924 771
- US-A- 4 587 964
- US-A- 5 089 003

## Description

### Domaine technique

La présente invention concerne un manche amovible muni d'un dispositif d'assemblage démontable et destiné à être monté sur un outil et plus particulièrement mais non exclusivement sur une râpe ou similaire utilisée notamment par un chirurgien pour calibrer ou usiner l'extrémité du fémur d'un patient lors de l'implantation d'une prothèse fémorale.

### Technique antérieure

Depuis déjà de nombreuses années, il est habituel de substituer à l'articulation, notamment du fémur sur le bassin, tout ou partie de cette articulation. On peut alors remplacer par une prothèse la tête du fémur ou le cotyle de l'acétabulum ou encore ces deux éléments d'articulation.

Pour la mise en place plus particulièrement d'une prothèse fémorale, le chirurgien doit tout d'abord choisir parmi une gamme de prothèses fémorales, la prothèse la plus adaptée à la morphologie du patient et notamment à la longueur du col fémoral. Pour ce choix, le chirurgien doit réaliser un certain nombre d'essais avant d'en sélectionner une et de la poser définitivement.

Ensuite, pour la mise en place de la prothèse retenue, après avoir supprimé la tête fémorale, le chirurgien doit aménager la cavité médullaire du fémur à l'aide d'une râpe chirurgicale ou similaire, dont la conformation est très proche de la tige de ladite prothèse qu'il devra engager dans le canal médullaire du fémur. Pour obtenir l'aménagement final, le chirurgien va utiliser successivement plusieurs râpes de forme homothétique dont les dimensions sont de plus en plus grandes, afin d'aménager la cavité médullaire en plusieurs étapes successives. Pour cela, le chirurgien solidarise successivement les différentes râpes à l'une des extrémités d'un manche amovible pour enfoncer chaque râpe dans le canal médullaire du fémur en tapant sur l'extrémité libre dudit manche, ce dernier étant muni en cette extrémité libre d'une tête de frappe et d'un dispositif d'assemblage démontable rapide du type à emboîtement selon une direction perpendiculaire au plan de joint entre le manche et la râpe permettant un verrouillage en translation de cet emboîtement pour éviter tout désaccouplement non volontaire. Par ailleurs, les dernières évolutions apportées aux prothèses de hanches, telles que les cols modulaires, imposent d'avoir des râpes fémorales munies d'un logement femelle apte à permettre l'emboîtement d'un manche amovible.

A cet égard, on connaît déjà de nombreux manches amovibles munis d'un dispositif d'assemblage démontable comportant un moyen d'emboîtement mâle. Toutefois, les manches amovibles connus sont pourvus d'un dispositif d'assemblage démontable ne permettant pas d'obtenir un blocage sans jeu entre lesdits manches et les râpes. Ce jeu résiduel a notamment pour effet d'autoriser une légère rotation et/ou une légère translation de la râpe par rapport au manche associé. Ces légers mouvements peuvent être préjudiciables à l'évaluation de la stabilité primaire de la râpe dans le fémur et donc à l'évaluation de la stabilité primaire de la prothèse. Ce jeu résiduel peut également apparaître au fil des jours à cause de l'usure des pièces et de leurs assemblages.

Enfin, comme le logement, notamment femelle, des râpes permettant l'emboîtement du manche amovible n'est pas totalement standardisé, chaque fabricant de prothèses a sa gamme de râpes avec son propre modèle de logement et propose donc de mettre à disposition du chirurgien avec sa gamme de râpes son propre manche amovible avec un dispositif d'assemblage démontable rapide compatible avec le logement de ses râpes. Par conséquent, lorsque le manche est cassé ou lorsque les râpes sont usées ou cassées, le chirurgien est obligé soit de rester avec le même fabricant de prothèses pour renouveler le manche ou sa gamme de râpes associés, soit d'acheter ses prothèses chez un autre fabricant pour obtenir une nouvelle gamme de râpes mais également un nouveau manche amovible car les manches ne sont a priori pas compatibles d'un fabricant de prothèses à l'autre.

Document US 4 587 964 divulgue les caractéristiques du préambule de la revendication 1.

### Exposé de l'invention

Le but de la présente invention est donc de pallier les inconvénients précédemment cités et de proposer un manche amovible muni d'un dispositif d'assemblage démontable et destiné à être monté sur un outil, du type râpe chirurgicale ou similaire, ledit dispositif d'assemblage garantissant un blocage sans jeu résiduel permettant une immobilisation en translation mais également en rotation du manche par rapport à la râpe, ledit dispositif d'assemblage démontable pouvant être rendu compatible, à moindre coût, avec une grande majorité des râpes munies d'une logement femelle disponibles sur le marché.

Conformément à l'invention, il est donc proposé un manche amovible pour un outil et plus particulièrement mais non exclusivement pour une râpe chirurgicale ou similaire, le manche et la râpe coopérant au droit de leurs extrémités d'assemblage par des faces de contact au moins sécantes à la direction moyenne de transmission des efforts entre le manche et la râpe. Ledit manche comportant un dispositif d'assemblage démontable est remarquable en ce que le dispositif d'assemblage démontable comporte, d'une part, un moyen d'assemblage par emboitement, selon une direction globalement perpendiculaire auxdites faces de contact, mettant en oeuvre au moins deux organes complémentaires à savoir un tenon creux et une mortaise respectivement solidaires du manche amovible et de la râpe et, d'autre part, un moyen de verrouillage desdits organes complémentaires en leur position emboitée c'est à dire lorsque le manche amovible et la râpe sont solidarisés, leurs faces de contact étant appliquées l'une contre l'autre, ledit moyen de verrouillage comportant au moins :
- un coulisseau mobile apte à coulisser au moins en partie à l'intérieur du tenon selon une direction sensiblement parallèle à la direction d'emboitement dudit moyen d'assemblage entre une position "assemblage verrouillé" dans laquelle il interdit tout démontage entre le manche amovible et la râpe et une position "assemblage libre" dans laquelle il permet une libération du manche,
- une entaille aménagée sur la mortaise,
- un organe de blocage disposé au moins en partie à l'intérieur du tenon et apte à coopérer simultanément avec le coulisseau et l'entaille lorsque que le tenon est emboîté dans la mortaise, et
- un levier de manoeuvre pivotant par rapport au manche entre au moins deux positions d'actionnement à savoir une position "levier fermé" et une position "levier ouvert" déterminant au moins indirectement respectivement les positions "assemblage verrouillé" et "assemblage libre" du coulisseau,
et en ce que les formes respectives du coulisseau et de l'entaille sont telles que, lorsque que le tenon du manche est emboîté dans la mortaise de la râpe et que le levier de manoeuvre est en position "levier fermé", l'organe de blocage s'écarte vers l'extérieur du tenon en prenant appui sur l'entaille de manière de générer un effort de serrage l'une contre l'autre des faces de contact respectives du manche et de la râpe.

De manière avantageuse, le moyen de verrouillage comporte au moins un poussoir articulé à chacune de ses extrémités au coulisseau et au levier de manoeuvre autour d'axes sensiblement parallèles à l'axe de rotation dudit levier de manoeuvre pour transmettre le mouvement du levier de manoeuvre au coulisseau.

L'ensemble levier de manoeuvre-poussoir-coulisseau est configuré pour être du type genouillère mécanique.

Le poussoir est avantageusement extensible. A cet égard, le poussoir comporte un ensemble vis-écrous à pas différentiel et/ou un organe élastique avantageusement du type rondelles ressorts, appelées rondelles "Belleville".

Le tenon comporte de préférence au moins un orifice traversant l'épaisseur dudit tenon, apte à recevoir l'organe de blocage et dimensionné de manière à permettre à ce dernier de dépasser uniquement en partie de la face externe du tenon tout en le retenant afin d'éviter qu'il ne s'échappe de l'orifice et dudit tenon.

De même, le coulisseau présente une tête globalement parallélépipédique dont la section longitudinale est en forme générale de trapèze de manière à avoir au moins une face latérale inclinée par rapport à la direction d'emboitement du moyen d'assemblage et en direction de l'extrémité libre du tenon, ladite face latérale comportant un canal longitudinal disposé en regard dudit orifice lorsque la tête est à l'intérieur dudit tenon et apte à coopérer avec l'organe de blocage.

De manière avantageuse, l'entaille est disposée en vis-à-vis dudit orifice lorsque le tenon est emboîté dans la mortaise et inclinée par rapport à la direction d'emboitement du moyen d'assemblage et en direction de l'extrémité libre de la mortaise, ladite entaille étant inclinée dans le sens opposé par rapport à l'inclinaison du canal associé.

Selon un mode de réalisation préféré, le moyen de verrouillage comporte deux organes de blocage et deux entailles aménagées en vis-à-vis sur la mortaise le long d'un axe sensiblement perpendiculaire à la direction d'emboitement du moyen d'assemblage et symétriquement par rapport à l'axe longitudinal de la mortaise, le tenon comporte deux orifices disposés en vis-à-vis le long d'un axe sensiblement perpendiculaire à la direction d'emboitement du moyen d'assemblage, et la tête a une section longitudinale en forme générale de trapèze régulier de manière à avoir deux faces latérales symétriquement inclinées par rapport à la direction d'emboitement du moyen d'assemblage et en direction de l'extrémité libre du tenon et comportant chacune un canal longitudinal.

Chaque organe de blocage est avantageusement une bille.

### Description sommaire des figures

D'autres avantages et caractéristiques ressortiront mieux de la description qui va suivre d'un mode d'exécution d'un manche amovible muni d'un dispositif d'assemblage démontable selon l'invention en référence aux figures annexées sur lesquelles :
- la figure 1 est une vue de face d'un manche muni d'un dispositif d'assemblage démontable selon l'invention associé à une râpe ;
- la figure 2 est une vue partielle du manche de la figure 1 avec le levier de manoeuvre en position "levier fermé" ;
- la figure 3 est une coupe verticale partielle agrandie d'un détail de la figure 2 ;
- la figure 4 est une vue en perspective partielle du manche semblable à celui de la figure 2 avec le levier de manoeuvre en position "levier ouvert" ;
- la figure 5 est une vue de détail agrandie de l'extrémité du manche de la figure 4 ;
- la figure 6 est une vue en perspective partielle du manche de la figure 2 ;
- la figure 7 est une vue de détail agrandie de l'extrémité du manche de la figure 6.

### Meilleure manière de réaliser l'invention technique

On décrira ci-après un manche amovible muni d'un dispositif d'assemblage démontable permettant le montage dudit manche sur un outil, du type râpe chirurgicale ou similaire. Il va de soi que ledit dispositif d'assemblage démontable pourra être utilisé pour solidariser sans jeu ledit manche sur un outil d'un tout autre type sans sortir du cadre de la présente invention.

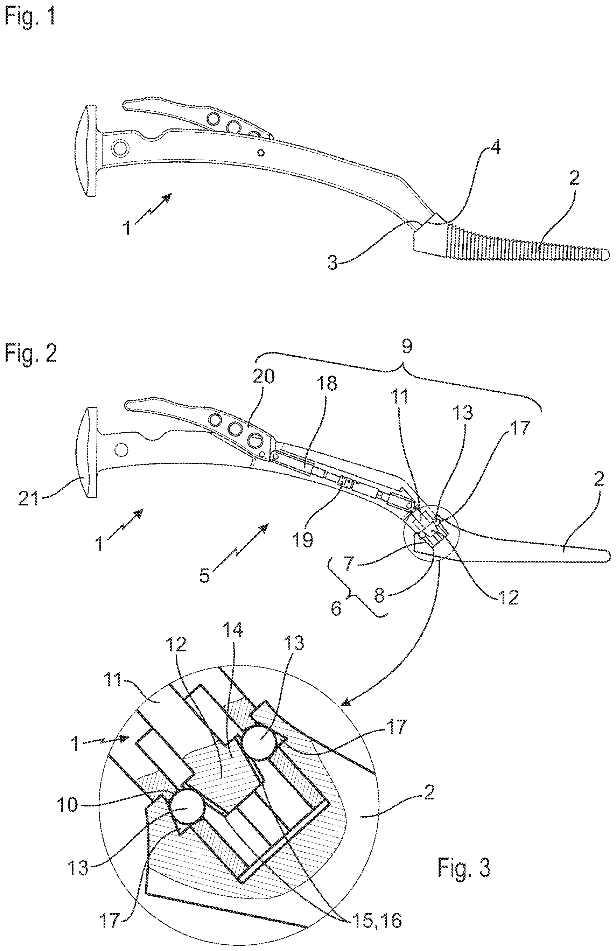

En référence à la figure 1, le manche 1 amovible est monté sur une râpe 2 utilisée notamment par un chirurgien pour calibrer ou usiner l'extrémité du fémur d'un patient lors de l'implantation d'une prothèse fémorale. Le manche 1 et la râpe 2 coopèrent au droit de leurs extrémités d'assemblage par des faces de contact respectivement 3, 4 au moins sécantes à la direction moyenne de transmission des efforts entre le manche 1 et la râpe 2.

En référence aux figures 2 à 7, le manche 1 amovible comporte un dispositif d'assemblage 5 démontable permettant le montage du manche 1 amovible sur la râpe 2.

Le dispositif d'assemblage 5 comprend, d'une part, un moyen d'assemblage 6 par emboitement, selon une direction globalement perpendiculaire aux faces de contact 3, 4 respectives du manche 1 et de la râpe 2, mettant en oeuvre au moins deux organes complémentaires à savoir un tenon 7 et une mortaise 8 et, d'autre part, un moyen de verrouillage 9 de ces organes complémentaires 7, 8 en leur position emboitée c'est-à-dire lorsque le manche 1 amovible et la râpe 2 sont solidarisés l'un avec l'autre.

Le tenon 7 et la mortaise 8 sont avantageusement de formes complémentaires globalement cylindriques ou coniques allongées, respectivement solidaires du manche 1 amovible et de la râpe 2 et s'étendant sensiblement perpendiculairement à leurs faces de contact 3, 4 respectives.

On désigne ici par forme cylindrique une forme dont la surface est définie par une droite, appelée génératrice, passant par un point variable décrivant une courbe plane fermée, appelée courbe directrice et gardant une direction fixe classiquement perpendiculaire au plan de la courbe directrice.

En outre, ledit tenon 7 est creux et comporte deux orifices 10 disposés en vis-à-vis le long d'un axe sensiblement perpendiculaire à la direction d'emboitement du moyen d'assemblage 6 et traversant entièrement l'épaisseur du tenon 7 de manière à permettre le passage de l'intérieur vers l'extérieur dudit tenon 7.

Ledit moyen de verrouillage 9 comporte un coulisseau 11 mobile présentant une tête 12 globalement parallélépipédique apte à coulisser à l'intérieur du tenon 7 selon une direction sensiblement perpendiculaire à la face de contact 3 du manche 1 et deux organes de blocage 13, avantageusement du type bille, disposés à l'intérieur du tenon 7. Ledit coulisseau 1 est mobile entre une position "assemblage verrouillé" dans laquelle la tête 12 interdit tout démontage entre le manche 1 amovible et la râpe 2 et une position "assemblage libre" dans laquelle la tête 12 permet une libération du manche 1.

Ladite tête 12 a une section longitudinale en forme générale de trapèze régulier de manière à avoir deux faces latérales 14 parallèles et deux faces latérales inclinées 15 par rapport à la direction d'emboitement du moyen d'assemblage 6 et en direction de l'extrémité libre du tenon 7, c'est-à-dire que plus on se place vers ladite extrémité libre plus la tête 12 est étroite. Ces dernières comportent chacune un canal longitudinal 16 apte à coopérer avec l'un des organes de blocage 13 et disposé en regard d'un des orifices 10 du tenon 7 lorsque la tête 12 est à l'intérieur dudit tenon 7.

Chaque orifice 10 du tenon 7 reçoit un organe de blocage 13 et est dimensionné de manière à permettre à ce dernier de dépasser uniquement en partie de la face externe du tenon 7 tout en le retenant afin d'éviter qu'il ne s'échappe de l'orifice 10 et dudit tenon 7.

Ledit moyen de verrouillage 9 comporte en outre deux entailles 17 aménagées sur la mortaise 8 de la râpe 2 disposées en vis-à-vis le long d'un axe sensiblement perpendiculaire à la direction d'emboitement du moyen d'assemblage 6, chaque entaille 17 étant disposée en vis-à-vis d'un des orifices 10 du tenon 7 du manche 1 lorsque ce dernier est emboîtée dans ladite mortaise 8. Chaque entaille 17 est de forme globalement cylindrique de révolution et inclinée par rapport à la direction d'emboitement du moyen d'assemblage 6 et en direction de l'extrémité libre de la mortaise 8, c'est-à-dire que plus on se place vers ladite extrémité libre plus l'entaille 17 est étroite. Ces entailles 17 sont symétriques par rapport à l'axe longitudinal de la mortaise 8 et inclinées dans le sens opposé par rapport à l'inclinaison du canal 16 associé de la tête 12 du coulisseau 11. Chaque entaille 17 est apte à coopérer avec un des organes de blocage 13 pour bloquer le moyen d'assemblage 6.

En référence aux figures 2, 3, 6 et 7, lorsque que le tenon 7 du manche 1 est emboîté dans la mortaise 8 de la râpe 2, chaque organe de blocage 13, qui est logé dans un orifice 10 du tenon 7, coopère simultanément avec un canal 16 de la tête 12 du coulisseau 11 et une entaille 17 de la mortaise 8. On comprend alors bien que, compte tenu de l'inclinaison du canal 16, lorsque le levier de manoeuvre 20 est refermé, le coulisseau 11 se déplace en direction de l'intérieur de la râpe 2 jusqu'à la position "assemblage verrouillé", les organes de blocage 13 s'écartent vers l'extérieur du tenon 7 en prenant appui sur l'entaille 17 associée. Compte tenu de l'inclinaison des entailles 17, cette action des organes de blocage 13 a pour effet de générer un effort de serrage l'une contre l'autre des faces de contact 4, 5 respectives du manche 1 et de la râpe 2. Cette particularité technique permet de garantir la non-démontabilité de l'assemblage manche 1-râpe 2 et surtout la suppression de tout jeu résiduel entre lesdits manche 1 et râpe 2.

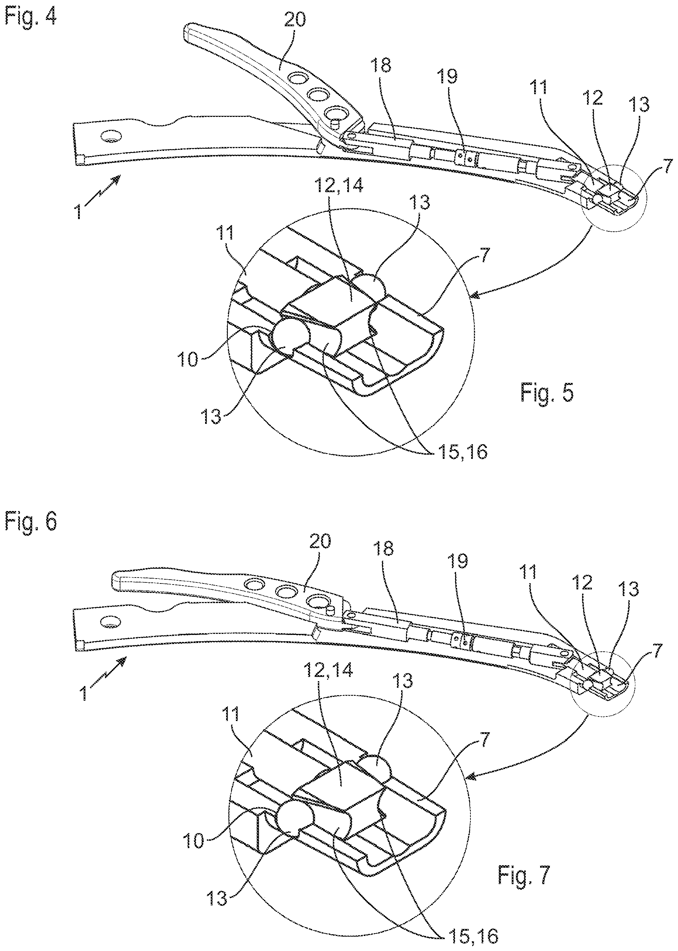

A l'inverse, en référence à la figure 5, pour pouvoir libérer le manche 1, il faut que le coulisseau 11 se déplace en direction de l'intérieur du manche 1 jusqu'à la position "assemblage libre" afin de permettre la rentrée totale des organes de blocage 13 à l'intérieur du tenon 7 et le désemboîtement du moyen d'assemblage 6 et donc du manche 1.

On comprend bien que l'inclinaison des canaux 16 et celle des entailles 17 pourront être inversées sans sortir du cadre de la présente invention. Ainsi, la tête 12 aura alors deux faces latérales inclinées 15 par rapport à la direction d'emboitement du moyen d'assemblage 6 et en direction de l'intérieur du tenon 7 et chaque entaille 17 sera inclinée par rapport à la direction d'emboitement du moyen d'assemblage 6 et en direction de l'intérieur de la mortaise 8. Avec une telle configuration, le coulisseau 11 se déplacera alors en direction de l'intérieur du manche 1 pour se déplacer de sa position "assemblage libre" à sa position "assemblage verrouillé".

En référence aux figures 2, 4 et 6, le dispositif d'assemblage 5 comprend au moins un poussoir 18 mobile entre deux positions dites positions utiles dont, d'une part, une position dans laquelle il coopère avec le coulisseau 11 pour précisément le contraindre en position "assemblage verrouillé" et, d'autre part, une autre position dans laquelle il place le coulisseau 11 en position "assemblage libre" de manière à permettre l'assemblage ou la séparation du manche 1 et de la râpe 2.

Ce poussoir 18 est extensible, c'est-à-dire que sa longueur est variable afin de permettre un réglage de la contrainte qu'il va exercer sur le coulisseau 11. Ce réglage permet, d'une part, d'avoir la valeur de contrainte la mieux adaptée et, d'autre part, de rattraper dans le temps le jeu résiduel lié notamment à l'usure des pièces constituant le dispositif d'assemblage 5. Pour ce faire, le poussoir 18 comporte de préférence un ensemble vis-écrous à pas différentiel 19 pour permettre une variation très petite de la longueur dudit poussoir 18 et par conséquent un réglage très précis de la contrainte appliquée.

Selon une variante de réalisation non représentée, le poussoir 18 comporte un organe élastique avantageusement du type rondelles ressorts, appelées communément rondelles "Belleville".

Selon une autre variante de réalisation non représentée, le poussoir 18 comporte un ensemble vis-écrous à pas différentiel 19 associé à un organe élastique, l'ensemble vis-écrous à pas différentiel 19 permettant le réglage précis et l'organe élastique permettant d'adoucir les manipulations et de compenser l'usure.

En référence aux figures 1, 2, 4 et 6, le dispositif d'assemblage 5 comprend au moins un levier de manoeuvre 20 pivotant par rapport au manche 1 autour d'un axe sensiblement perpendiculaire au plan longitudinal de symétrie de l'ensemble manche 1-râpe 2 de poussoir 18 entre au moins deux positions d'actionnement à savoir une position "levier fermé" dans laquelle le levier de manoeuvre 20 s'étend sensiblement parallèlement et le long du manche 1 sur lequel il s'articule (Cf. figures 1, 2 et 6) et une position "levier ouvert" dans laquelle ledit levier de manoeuvre 20 forme un certain angle avec ledit manche 1 (Cf. figure 4). Les positions "levier fermé" et "levier ouvert" déterminent au moins indirectement respectivement les positions "assemblage verrouillé" et "assemblage libre" du coulisseau 11.

Pour cela, le poussoir 18 est articulé à chacune de ses extrémités au coulisseau 11 et au levier de manoeuvre 20 autour d'axes sensiblement parallèles à l'axe de rotation dudit levier de manoeuvre 20 par rapport au manche 1 de sorte que la rotation de ce dernier, par une action développée notamment par fermeture de la main sur le manche 1, entraîne le coulissement du coulisseau 11 en partie à l'intérieur du tenon 7 suivant la direction d'emboîtement du moyen d'assemblage 6 pour bloquer le manche 1 sur la râpe 2. Le poussoir 18 fait donc office de biellette pour transmettre le mouvement du levier de manoeuvre 20 au coulisseau 11.

Par ailleurs, l'ensemble levier de manoeuvre 20-poussoir 18-coulisseau 11 est configuré pour être du type genouillère mécanique, c'est-à-dire que le levier de manoeuvre 20 se bloque lorsque l'axe d'articulation entre le levier de manoeuvre 20 et le poussoir 18 a dépassé le point d'alignement entre les trois axes d'articulation et arrive en butée, les trois axes d'articulation correspondant aux deux axes d'articulation du poussoir 18 sur le coulisseau 11 et sur levier de manoeuvre 20 et à l'axe de rotation dudit levier de manoeuvre 20 sur le manche 1, ladite position bloquée correspondant à la position "levier fermé" du levier de manoeuvre 20.

Ainsi avec une telle configuration, lorsque le levier de manoeuvre 20 est en position "levier fermé", il reste, sans l'intervention d'un utilisateur, dans cette position (Cf. figure 2),.

Cette particularité technique permet de sécuriser l'assemblage entre la manche 1 et la râpe 2 en évitant toute ouverture intempestive du levier de manoeuvre 20 de manoeuvre.

Parmi les différents modèles de râpe 2, les formes et dimensions de la mortaise 8 sont relativement standardisées, à peine plus de trois ou quatre modèles de mortaises, de sorte qu'en fabriquant trois ou quatre modèles de manches 1 avec un tenon 7 correspondant aux différents modèles de mortaises 8, on pourra fournir à moindre coût une gamme de manches 1 compatibles avec toutes les râpes 2 du marché. En effet, pour rendre ces manches 1 totalement compatibles il faudra juste réaliser sur chaque râpe 2 les entailles 17. Chaque fabricant de prothèses et de râpes 2 associées pourra donc rendre, facilement et à moindre coût, ses râpes compatibles avec le manche 1 selon l'invention.

Par ailleurs, le manche 1 selon l'invention pourra ne comporter qu'un seul orifice 10, qu'un seul organe de blocage 13, qu'un seul canal 16 et qu'une seule entaille 17 sans sortir de la présente invention.

Enfin, le manche 1 comporte une zone de frappe 21 (représentée sur les figures 1 et 2) à son extrémité libre pour permettre au chirurgien d'enfoncer chaque râpe dans le canal médullaire du fémur en tapant sur ladite zone de frappe 21.

### Possibilité d'application industrielle

Le manche 1 selon l'invention s'applique plus particulièrement aux râpes 2 fémorales ou similaires, mais il peut également être utilisé pour un tout autre outil dont on souhaite interdire tout jeu résiduel lors de son assemblage avec ledit manche 1.

Enfin, il va de soi que les exemples de manches 1 conformes à l'invention qui viennent d'être décrits ne sont que des illustrations particulières, en aucun cas limitatives de l'invention.

## Revendications

1. Manche (1) amovible pour un outil et plus particulièrement mais non exclusivement pour une râpe (2) chirurgicale ou similaire, le manche (1) et la râpe (2) coopérant au droit de leurs extrémités d'assemblage par des faces de contact respectivement (3, 4) au moins sécantes à la direction moyenne de transmission des efforts entre le manche (1) et la râpe (2), ledit manche (1) comportant un dispositif d'assemblage (5) démontable qui comporte, d'une part, un moyen d'assemblage (6) par emboitement, selon une direction globalement perpendiculaire auxdites faces de contact (3, 4), mettant en oeuvre au moins deux organes complémentaires à savoir un tenon (7) creux et une mortaise (8) respectivement solidaires du manche (1) amovible et de la râpe (2) et, d'autre part, un moyen de verrouillage (9) desdits organes complémentaires (7, 8) en leur position emboitée c'est à dire lorsque le manche 1 amovible et la râpe (2) sont solidarisés, leurs faces de contact (3, 4) étant appliquées l'une contre l'autre, ledit moyen de verrouillage (9) comportant au moins :
- un coulisseau (11) mobile apte à coulisser au moins en partie à l'intérieur du tenon (7) selon une direction sensiblement parallèle à la direction d'emboitement dudit moyen d'assemblage (6) entre une position "assemblage verrouillé" dans laquelle il interdit tout démontage entre le manche (1) amovible et la râpe (2) et une position "assemblage libre" dans laquelle il permet une libération du manche (1),
- une entaille (17) aménagée sur la mortaise (8),
- un organe de blocage (13) disposé au moins en partie à l'intérieur du tenon (7) et apte à coopérer simultanément avec le coulisseau (11) et l'entaille (17) lorsque que le tenon (7) est emboîté dans la mortaise (8), et étant **caractérise en ce que** le moyen de verrouillage comporte
- un levier de manoeuvre (20) pivotant par rapport au manche (1) entre au moins deux positions d'actionnement à savoir une position "levier fermé" et une position "levier ouvert" déterminant au moins indirectement respectivement les positions "assemblage verrouillé" et "assemblage libre" du coulisseau (11),
et **en ce que** les formes respectives du coulisseau (11) et de l'entaille (17) sont telles que, lorsque que le tenon 7 du manche 1 est emboîté dans la mortaise 8 de la râpe (2) et que le levier de manoeuvre (20) est en position "levier fermé", l'organe de blocage (13) s'écarte vers l'extérieur du tenon (7) en prenant appui sur l'entaille (17) de manière de générer un effort de serrage l'une contre l'autre des faces de contact (3, 4) respectives du manche (1) et de la râpe (2).

2. Manche (1) selon la revendication 1, **caractérisé en ce que** le moyen de verrouillage (9) comporte au moins un poussoir (18) articulé à chacune de ses extrémités au coulisseau (11) et au levier de manoeuvre (20) autour d'axes sensiblement parallèles à l'axe de rotation dudit levier de manoeuvre (20) pour transmettre le mouvement du levier de manoeuvre (20) au coulisseau (11).

3. Manche (1) selon la revendication 2, **caractérisé en ce que** l'ensemble levier de manoeuvre (20)-poussoir (18)-coulisseau (11) est configuré pour être du type genouillère mécanique.

4. Manche (1) selon l'une quelconque des revendications 2 ou 3, **caractérisé en ce que** le poussoir (18) est extensible.

5. Manche (1) selon la revendication 4, **caractérisé en ce que** le poussoir (18) comporte un ensemble vis-écrous à pas différentiel (19) et/ou un organe élastique du type rondelles ressorts, appelées rondelles "Belleville".

6. Manche (1) selon l'une quelconque des revendications 1 à 5, **caractérisé en ce que** le tenon (7) comporte au moins un orifice (10) traversant l'épaisseur dudit tenon (7), apte à recevoir l'organe de blocage (13) et dimensionné de manière à permettre à ce dernier de dépasser uniquement en partie de la face externe du tenon (7) tout en le retenant afin d'éviter qu'il ne s'échappe de l'orifice (10) et dudit tenon (7).

7. Manche (1) selon la revendication 6, **caractérisé en ce que** le coulisseau (11) présente une tête (12) globalement parallélépipédique dont la section longitudinale est en forme générale de trapèze de manière à avoir au moins une face latérale inclinée (15) par rapport à la direction d'emboitement du moyen d'assemblage (6) et en direction de l'extrémité libre du tenon (7), ladite face latérale inclinée (15) comportant un canal (16) longitudinal disposé en regard dudit l'orifice (10) lorsque la tête (12) est à l'intérieur dudit tenon (7) et apte à coopérer avec l'organe de blocage (13).

8. Manche (1) selon la revendication 7, **caractérisé en ce que** l'entaille (17) est disposée en vis-à-vis dudit orifices (10) lorsque le tenon (7) est emboîté dans la mortaise (8) et inclinée par rapport à la direction d'emboitement du moyen d'assemblage (6) et en direction de l'extrémité libre de la mortaise (8), ladite entaille (17) étant inclinée dans le sens opposé par rapport à l'inclinaison du canal (16) associé.

9. Manche (1) selon la revendication 8, **caractérisé en ce que** le moyen de verrouillage (9) comporte deux organes de blocage (13) et deux entailles (17) aménagées sur la mortaise (8) en vis-à-vis le long d'un axe sensiblement perpendiculaire à la direction d'emboitement du moyen d'assemblage (6) et symétriquement par rapport à l'axe longitudinal de la mortaise (8), **en ce que** le tenon (7) comporte deux orifices (10) disposés en vis-à-vis le long d'un axe sensiblement perpendiculaire à la direction d'emboitement du moyen d'assemblage (6), et **en ce que** la tête (12) a une section longitudinale en forme générale de trapèze régulier de manière à avoir deux faces latérales inclinées (15) symétriquement par rapport à la direction d'emboitement du moyen d'assemblage (6) et en direction de l'extrémité libre du tenon (7) et comportant chacune un canal (16) longitudinal.

10. Manche (1) selon l'une quelconque des revendications 1 à 9, **caractérisé en ce que** l'organe de blocage (13) est une bille.

## Patentansprüche

1. Abnehmbarer Griff (1) für ein Werkzeug, und insbesondere, jedoch nicht ausschließlich für eine chirurgische Raspel (2) oder ähnliches, wobei der Griff (1) und die Raspel (2) auf Höhe ihrer Montageenden durch jeweils zumindest in die mittlere Übertragungsrichtung der Kräfte zwischen dem Griff (1) und der Raspel (2) schneidenden Kontaktflächen (3, 4) zusammenwirken, wobei der besagte Griff (1) eine demontierbare Montagevorrichtung (5) umfasst, die einerseits ein Montagemittel (6) durch Einstecken in eine Richtung umfasst, die im Allgemeinen senkrecht zu den besagten Kontaktflächen (3, 4) verläuft, welches zumindest zwei einander ergänzende Organe, nämlich einen hohlen Zapfen (7) und ein Zapfenloch (8) verwendet, die jeweils fest mit dem abnehmbaren Griff (1) und der Raspel (2) verbunden sind, und andererseits ein Verriegelungsmittel (9) für die besagten einander ergänzenden Organe (7, 8) in ihrer eingesteckten Position, das heißt, wenn der abnehmbare Griff (1) und die Raspel (2) fest miteinander verbunden sind, ihre Kontaktflächen (3, 4) aneinander angelegt sind, wobei das besagte Verriegelungsmittel (9) zumindest Folgendes umfasst:
- einen beweglichen Schieber (11), der imstande ist, zumindest teilweise im Inneren des Zapfens (7) in eine Richtung im Wesentlichen parallel zur Einsteckrichtung des besagten Montagemittels (6) zwischen einer "verriegelten Montage" Position, in der er jedwede Demontage zwischen dem abnehmbaren Griff (1) und der Raspel (2) untersagt, und einer "freien Montage" Position zu gleiten, in der er eine Freigabe des Griffs (1) erlaubt,
- eine Kerbe (17), die am Zapfenloch (8) eingearbeitet ist,
- ein Sperrorgan (13), das zumindest teilweise im Inneren des Zapfens (7) angeordnet ist, und imstande ist, zugleich mit dem Schieber (11) und mit der Kerbe (17) zusammenzuwirken, wenn der Zapfen (7) im Zapfenloch (8) eingesteckt ist, und **dadurch gekennzeichnet, dass** das Verriegelungsmittel Folgendes umfasst
- einen Betätigungshebel (20), der im Verhältnis zum Griff (1) zwischen zumindest zwei Betätigungspositionen geschwenkt werden kann, nämlich einer Position für "Hebel geschlossen" und einer Position für "Hebel offen", die zumindest indirekt jeweils die Positionen der "verriegelten Montage" und der "freien Montage" des Schiebers (11) bestimmen,
und dadurch, dass die jeweiligen Formen des Schiebers (11) und der Kerbe (17) dergestalt sind, dass, wenn der Zapfen (7) des Griffs (1) in das Zapfenloch (8) der Raspel (2) eingesteckt ist, und sich der Betätigungshebel (20) in der Position für "Hebel geschlossen" befindet, sich das Sperrorgan (13) in Richtung Außenseite des Zapfens (7) abspreizt, sich an der Kerbe (17) anlegt, um eine Klemmkraft der jeweiligen Kontaktflächen (3, 4) des Griffs (1) und der Raspel (2) zueinander zu erzeugen.

2. Griff (1) nach Anspruch 1, **dadurch gekennzeichnet, dass** das Verriegelungsmittel (9) zumindest einen Stößel (18) umfasst, der an jedem seiner Enden am Schieber (11) und am Betätigungshebel (20) um Achsen gelenkig ist, die im Wesentlichen parallel zur Drehachse des besagten Betätigungshebels (20) sind, um die Bewegung des Betätigungshebels (20) auf den Schieber (11) zu übertragen.

3. Griff (1) nach Anspruch 2, **dadurch gekennzeichnet, dass** die Einheit Betätigungshebel (20)-Stößel (18)-Schieber (11) konfiguriert ist, um einen mechanischen Gelenkantrieb zu bilden.

4. Griff (1) nach einem der Ansprüche 2 oder 3, **dadurch gekennzeichnet, dass** der Stößel (18) erweiterbar ist.

5. Griff (1) nach Anspruch 4, **dadurch gekennzeichnet, dass** der Stößel (18) eine Einheit Schrauben-Muttern mit Differenzgewinde (19) und/oder ein elastisches Organ in der Art von Federringen umfasst, die "Tellerfedern" genannt werden.

6. Griff (1) nach einem der Ansprüche 1 bis 5, **dadurch gekennzeichnet, dass** der Zapfen (7) zumindest eine Öffnung (10) umfasst, die durch die Dicke des besagten Zapfens (7) hindurchführt, die imstande ist, das Sperrorgan (13) aufzunehmen, und bemessen ist, um es letzterem zu ermöglichen, nur teilweise über die Außenfläche des Zapfens (7) überzustehen, und ihn zurückzuhalten, um zu vermeiden, dass es aus der Öffnung (10) und dem besagten Zapfen (7) entweicht.

7. Griff (1) nach Anspruch 6, **dadurch gekennzeichnet, dass** der Schieber (11) einen im Allgemeinen quaderförmigen Kopf (12) aufweist, dessen Längsquerschnitt eine allgemeine Trapezform aufweist, um zumindest eine im Verhältnis zur Einsteckrichtung des Montagemittels (6) und in Richtung des freien Endes des Zapfens (7) geneigte Seitenfläche (15) aufzuweisen, wobei die besagte geneigte Seitenfläche (15) einen Längskanal (16) umfasst, der gegenüber der besagten Öffnung (10) angeordnet ist, wenn sich der Kopf (12) im Inneren des besagten Zapfens (7) befindet und imstande ist, mit dem Sperrorgan (13) zusammenzuwirken.

8. Griff (1) nach Anspruch 7, **dadurch gekennzeichnet, dass** die Kerbe (17) gegenüber den besagten Öffnungen (10) angeordnet ist, wenn der Zapfen (7) in das Zapfenloch (8) eingesteckt ist, und im Verhältnis zur Einsteckrichtung des Montagemittels (6) und in Richtung des freien Endes des Zapfenloches (8) geneigt ist, wobei die besagte Kerbe (17) in die entgegengesetzte Richtung im Verhältnis zur Neigung des zugehörigen Kanals (16) geneigt ist.

9. Griff (1) nach Anspruch 8, **dadurch gekennzeichnet, dass** das Verriegelungsmittel (9) zwei Sperrorgane (13) und zwei Kerben (17) umfasst, die einander gegenüber auf dem Zapfenloch (8) entlang einer Achse angeordnet sind, die im Wesentlichen senkrecht zur Einsteckrichtung des Montagemittels (6) und symmetrisch im Verhältnis zur Längsachse des Zapfenlochs (8) liegt, dadurch, dass der Zapfen (7) zwei Öffnungen (10) umfasst, die einander gegenüber, entlang einer Achse angeordnet sind, die im Wesentlichen senkrecht zur Einsteckrichtung des Montagemittels (6) liegt, und dadurch, dass der Kopf (12) einen Längsquerschnitt in einer allgemeinen regelmäßigen Trapezform aufweist, um zwei symmetrisch geneigte Seitenflächen (15) im Verhältnis zur Einsteckrichtung des Montagemittels (6) und in Richtung des freien Endes des Zapfens (7) aufzuweisen, und jeweils einen Längskanal (16) umfassend.

10. Griff (1) nach einem der Ansprüche 1 bis 9, **dadurch gekennzeichnet, dass** das Sperrorgan (13) eine Kugel ist.

## Claims

1. Removable handle (1) for a tool and more specifically but not exclusively for a surgical rasp (2) or the like, the handle (1) and the rasp (2) cooperating at right angles to their assembly ends by respective contact faces (3, 4) which at least intersect the mean direction of transmission of forces between the handle (1) and the rasp (2), said handle (1) comprising a device (5) for detachable assembly that has, on the one hand, means (6) for assembly by fitting, in a direction generally perpendicular to said contact faces (3, 4), implementing at least two complementary members, namely a hollow tenon (7) and a mortise (8), respectively secured to the removable handle (1) and the rasp (2) and, on the other hand, means (9) for locking said complementary members (7, 8) in their fitted position, i.e. when the removable handle (1) and the rasp (2) are secured, their contact faces (3, 4) being applied one against the other, said locking means (9) comprising at least:
- a mobile slide (11) capable of sliding at least partially inside the tenon (7) in a direction substantially parallel to the direction of fitting of said assembly means (6) between a "locked assembly" position in which it prevents any detachment between the removable handle (1) and the rasp (2) and a "free assembly" position in which it enables the handle (1) to be released,
- a notch (17) formed on the mortise (8),
- a blocking member (13) arranged at least partially inside the tenon (7) and capable of cooperating simultaneously with the slide (11) and the notch (17) when the tenon (7) is fitted in the mortise (8), and being **characterized in that** the locking means comprise:
- a maneuvering lever (20) pivoting with respect to the handle (1) between at least two actuation positions, namely a "closed lever" position and an "open lever" position at least indirectly determining, respectively, the "locked assembly" and "free assembly" positions of the slide (11),
and **in that** the respective shapes of the slide (11) and the notch (17) are such that, when the tenon (7) of the handle (1) is fitted in the mortise (8) of the rasp (2) and the maneuvering lever (20) is in the "closed lever" position, the blocking member (13) moves away toward the outside of the tenon (7) while bearing on the notch (17) so as to generate a clamping force, one against the other, of the respective contact faces (3, 4) of the handle (1) and the rasp (2).

2. Handle (1) according to claim 1, **characterized in that** the locking means (9) comprise at least one push-member (18) articulated at each of its ends to the slide (11) and to the maneuvering lever (20) around axes substantially parallel to the axis of rotation of said maneuvering lever (20) in order to transmit the movement of the maneuvering lever (20) to the slide (11).

3. Handle (1) according to claim 2, **characterized in that** the maneuvering lever (20) - push-member (18) - slide (11) assembly is configured in the form of a mechanical swivel.

4. Handle (1) according to any one of claims 2 or 3, **characterized in that** the push-member (18) is extendable.

5. Handle (1) according to claim 4, **characterized in that** the push-member (18) has a screw - nut assembly with a differential pitch (19) and/or a resilient member of the spring washer type, called "Belleville" washer.

6. Handle (1) according to any one of claims 1 to 5, **characterized in that** the tenon (7) has at least one hole (10) passing through the thickness of said tenon (7), capable of receiving the blocking member (13) and sized so as to enable the latter to go only partially beyond the outer face of the tenon (7) while retaining it so as to prevent it from completely emerging from the hole (10) and said tenon (7).

7. Handle (1) according to claim 6, **characterized in that** the slide (11) has a generally parallelepiped head (12), the longitudinal cross-section of which is generally trapezoidal so as to have at least one lateral face (15) inclined with respect to the direction of fitting of the assembly means (6) and in the direction of the free end of the tenon (7), said lateral inclined face (15) comprising a longitudinal channel (16) arranged opposite said hole (10) when the head (12) is inside said tenon (7) and capable of cooperating with the blocking member (13).

8. Handle (1) according to claim 7, **characterized in that** the notch (17) is arranged opposite said hole (10) when the tenon (7) is fitted in the mortise (8) and inclined with respect to the direction of fitting of the assembly means (6) and in the direction of the free end of the mortise (8), said notch (17) being inclined in the opposite direction with respect to the inclination of the associated channel (16).

9. Handle (1) according to claim 8, **characterized in that** the locking means (9) comprise two blocking members (13) and two notches (17) formed opposite one another on the mortise (8) along an axis substantially perpendicular to the direction of fitting of the assembly means (6) and symmetrically with respect to the longitudinal axis of the mortise (8), **in that** the tenon (7) comprises two holes (10) arranged opposite one another along an axis substantially perpendicular to the direction of fitting of the assembly means (6), and **in that** the head (12) has a longitudinal cross-section with a general regular trapezoid shape so as to have two lateral faces (15) symmetrically inclined with respect to the direction of fitting of the assembly means (6) and in the direction of the free end of the tenon (7) and each comprising a longitudinal channel (16).

10. Handle (1) according to any one of claims 1 to 9, **characterized in that** the blocking member (13) is a ball.
